Europäisches Patentamt

(19) European Patent Office    (11) Publication number: **0 064 986**

Office européen des brevets    **B1**

(12)    **EUROPEAN PATENT SPECIFICATION**

(45) Date of publication of patent specification: **14.08.85**    (51) Int. Cl.⁴: **C 07 C 51/56**

(21) Application number: **81902715.2**

(22) Date of filing: **24.09.81**

(86) International application number:
**PCT/US81/01289**

(87) International publication number:
**WO 82/01704 27.05.82 Gazette 82/14**

## (54) PREPARATION OF ACETIC ANHYDRIDE.

(30) Priority: **21.11.80 US 209350**

(43) Date of publication of application:
**24.11.82 Bulletin 82/47**

(45) Publication of the grant of the patent:
**14.08.85 Bulletin 85/33**

(84) Designated Contracting States:
**DE FR GB NL**

(56) References cited:
**BE-A- 819 455**
**GB-A-1 468 940**
**GB-A-2 013 184**
**GB-A-2 055 809**
**JP-A-77 003 926**
**US-A-3 579 566**
**US-A-4 046 807**
**US-A-4 252 741**
**ZA-A- 761 482**

**Derwent Abstracts, Showa Donko KK, "Acetic anhydride from methyl acetate and carbon monoxide-by contacting in presence of rhodium catalyst"**

(73) Proprietor: **EASTMAN KODAK COMPANY**
**343 State Street**
**Rochester New York 14650 (US)**

(72) Inventor: **LARKINS, Thomas Hassell**
**Rt. 8, 4408 Beechcliff Drive**
**Kingsport, TN 37664 (US)**
Inventor: **POLICHNOWSKI, Stanley Whitmore**
**547 Rambling Road**
**Kingsport, TN 37663 (US)**
Inventor: **TUSTIN, Gerald Charles**
**4052 Lakota Place**
**Kingsport, TN 37664 (US)**
Inventor: **YOUNG, David Alexander**
**1621 Sherwood Forest Boulevard**
**Baton Rouge, LA 70815 (US)**

(74) Representative: **Baron, Paul Alexander Clifford et al**
**Kodak Limited Patent Department Headstone Drive**
**Harrow Middlesex HA1 4TY (GB)**

(56) References cited:
**Derwent Abstracts, Showa Denko KK, "Acetic anhydride manufactured from methyl acetate and carbon monoxide-in presence of rhodium catalyst"**
**Chem Tech, October 1971, "Low pressure process for acetic acid via carbonylation", page 600, Roth et al**

## Description

This invention relates to a process for the preparation of acetic anhydride. More specifically, this invention relates to a liquid phase process for preparing acetic anhydride by carbonylating methyl acetate in the presence of hydrogen and a catalytic combination of rhodium, an iodine compound and lithium.

It is known that acetic anhydride, a very useful industrial chemical, can be prepared by the carbonylation of methyl acetate. It is also known that the formation of an undesirable by-product, often called "tar" or "soot" is virtually unavoidable in such a carbonylation reaction. Typically, this tar is formed in the preparation of acetic anhydride by the liquid phase carbonylation of methyl acetate in the presence of rhodium and an iodine compound at elevated temperature and pressure wherein a feed mixture containing methyl acetate is continuously fed to a reaction zone and a mixture containing acetic anhydride is continuously removed. In such a reaction system, tar formation can increase to the point where catalytic activity is greatly diminished and eventually results in termination of the carbonylation reaction.

Available analytical data do not permit absolute identification of the aforementioned tar. Based on combined information of IR (Infrared), H NMR (Hydrogen Nuclear Magnetic Resonance), C NMR (Carbon Nuclear Magnetic Resonance) and elemental analysis, certain aspects of the structure may be proposed; but the poor resolution in the H and C NMR spectra thwart hopes for absolute identification and are suggestive of a highly amorphous material. The C NMR shows two major broad band absorptions of approximately equal intensity, one in the alkyl region (13—45 $\delta$) and the other in the aromatic region (120—140 $\delta$). For the same material, the H NMR shows almost no aromatic protons relative to the alkyl bands at .9—1.7 and 1.6—3.0 $\delta$. In combination, these two spectra suggest a polyalkylated aromatic material substituted to the exclusion of aromatic protons. Additionally, the C NMR shows some very minor absorptions assignable to carbonyl moieties, a feature strongly suggested by the IR band at 1700 cm$^{-1}$. The IR also suggests carbon-oxygen (1180 cm$^{-1}$) and oxygen-hydrogen (36—3300 cm$^{-1}$) bonds. The elemental analysis substantiates the expectations of aromatic unsaturation showing .60 unsaturations per carbon and an empirical formula of $C_{36}H_{41}O_{4.15}$.

Unfortunately, tar formed in the carbonylation of methyl acetate, as previously described herein, increases as reaction conditions, such as temperature and pressure are increased to obtain acceptable space-time yields of acetic anhydride. Space-time yield is the yield of desired product in a unit of time per unit volume of reaction space. It is conveniently expressed in terms of gram/liter/hour(s). It is evident that a carbonylation process of the type described herein, which would combine high space-time yield of acetic anhydride, e.g. a space-time yield of 400 g./l./hr, with suppression of tar formation would represent a significant advance in the art.

The use of catalytic combinations or systems comprising rhodium and an iodine compound in the preparation of acetic anhydride by the carbonylation of methyl acetate has been reported in the patent literature. See, for example, BE—A—819,455 and JP—A—75—47922. These publications disclose that the reaction rate can be increased if the catalytic combination contains a promoter such as lithium. In such a process, the lithium is generally added as lithium iodide or lithium acetate.

For purposes of this invention, it is significant to note that neither BE—A—819,455 nor JP—A—75—47922 disclose including hydrogen gas in the carbonylation reaction.

US—A—4046807 discloses that tar formation can be suppressed by the inclusion of hydrogen in the gas fed to a carbonylation reactor for preparing acetic anhydride from methyl acetate using a rhodium-containing catalyst with a triphenyl phospine promoter. There is no disclosure in the patent of using a lithium promoter. Also, Example 13 of the patent clearly indicates that the use of hydrogen gas, as described therein, has a significant detrimental effect upon the yield of acetic anhydride.

EP—A—0008396 discloses that specific combinations of aliphatic carboxylic acids with heterocyclic quaternary ammonium compounds can be used to increase the catalytic activity of Group VIII noble metal-iodine compound compositions in the carbonylation of ethyl acetate. This publication also discloses that up to 10 volume percent of hydrogen gas can be included in the feed gas stream. There is no disclosure of using a lithium promoter in the catalyst system described or of using hydrogen gas in a critical concentration in a carbonylation process to both suppress tar formation and increase the yield of acetic anhydride.

GB—A—2013184 discloses the production of vinyl acetate by a process which includes a step wherein methyl acetate is carbonylated to acetic anhydride. The carbonylation reaction is conducted in the presence of a Group VIII noble metal (e.g., rhodium), carbon monoxide, a promoter (e.g., lithium), an inorganic co-promoter (e.g., a trivalent nitrogen or phosphorus compound), and a halide source (e.g., methyl iodide). It is disclosed that the carbon monoxide should be employed in substantially pure form but that inert diluents can be present. In particular, it is disclosed that hydrogen is not objectionable if present in only very small (trace) amounts as an impurity.

In accordance with the present invention there is provided a liquid-phase process for the preparation of acetic anhydride by the carbonylation of methyl acetate in the presence of rhodium, lithium, and an iodine compound, characterized in that hydrogen gas is also fed to the reaction zone in an amount of 2 to 7 volume percent, based on carbon monoxide and hydrogen gases.

It has been discovered that the use of 2—7 volume percent hydrogen, together with the catalytic combination of rhodium an iodine compound and lithium, as described herein, suppresses tar formation

3

and also significantly increases the reaction rate both in terms of methyl acetate conversion and acetic anhydride production.

The role of hydrogen in this process is not completely understood but it has been examined by infrared spectroscopic monitoring of an ongoing reaction. A batch autoclave coupled with a high-temperature, high-pressure infrared cell, was used to observe the rhodium complexes present during the reaction. In the absence of hydrogen feed the major rhodium species in solution at 175°C. and 800 psig (5616 kPa) is *trans*-$Rh(CO)_2I_4^\ominus$ as evidenced by the metal carbonyl absorption at 2090 $cm^{-1}$. (The pressures indicated in kPa herein are absolute pressures rather than gauge pressures). A smaller amount of *cis*-$Rh(CO)_2I_2^\ominus$ is also present under these conditions as indicated by the absorptions at 2056 and 1983 $cm^{-1}$ for the carbonyls bonded to rhodium. The addition of hydrogen to the reaction at 175°C. and 800 psig (5616 kPa) results in the rapid disappearance of the infrared absorption at 2090 $cm^{-1}$ for *trans*-$Rh(CO)_2I_4^\ominus$. At the same time, the absorptions for *cis*-$Rh(CO)_2I_2^\ominus$ at 2056 and 1983 $cm^{-1}$ dramatically increase in intensity. The observed transformation of *trans*-$Rh(CO)_2I_4^\ominus$ to *cis*-$Rh(CO)_2I_2^\ominus$ is also accompanied by a substantial increase in the rate of formation of acetic anhydride. On the basis of the infrared spectroscopic measurements it appears that the addition of hydrogen in the process of this invention increases the concentration of active catalyst, *cis*-$Rh(CO)_2I_2^\ominus$.

In practicing this invention, the minimum amount of hydrogen that gives a significant effect is 2 volume percent based on the total carbon monoxide and hydrogen introduced to the reaction zone. The use of greater than 7 volume percent hydrogen does not significantly improve either the suppression of tar formation or the rate of acetic anhydride formation. The preferred amount of hydrogen used in practicing this invention is 3 to 6 percent, based on total amount of hydrogen and carbon monoxide fed to the carbonylation reaction zone.

In a typical practice of the process, the feed to the carbonylation reactor is such as to maintain within the reaction mixture (1) 250 to 1300 ppm, preferably 500 to 1000 ppm, Rh, (2) 175 to 5000 ppm, preferably 1500 to 3700 ppm, lithium and (3) 7 to 35 weight percent methyl iodide. The remainder of the reactor contents is mostly methyl acetate reactant and acetic anhydride product with minor amounts of by-products such as ethylidene diacetate and acetone. The reactor feed optionally may contain a solvent such as acetic acid, e.g. in an amount that will maintain 5 to 40 weight percent in the reaction mixture. When reaction product is withdrawn in liquid form, the catalyst components, i.e. the rhodium, lithium and iodine as methyl iodide, are recovered from the reactor effluent and are recycled. When necessary, fresh rhodium, as rhodium chloride, rhodium acetate or other rhodium containing compound, and lithium, as lithium hydroxide, lithium iodide, lithium acetate or other lithium-containing compound are added to the catalyst recycle. The fresh rhodium and lithium can be conveniently added as a solution in acetic acid. When the iodine needs to be supplemented it may be added to the system as iodine ($I_2$), as methyl iodide or, at least in part, as lithium iodide. When reaction product is withdrawn in vapour form, all or essentially all of the rhodium and lithium catalyst components remain in the reactor and, the risk of their depletion from the process is reduced considerably.

The methyl acetate fed to the reactor consists primarily of fresh methyl acetate, which should be essentially anhydrous, and some recycled material. The feed may also contain recycled acetic anhydride although it is advantageous to add methanol to any recycled anhydride to convert the latter to methyl acetate feedstock.

The process may be carried out at elevated temperatures and pressures in the range of 160 to 220°C. and 300 to 1200 psig. (2170 and 8375 kPa). The particular temperature and pressure employed depends on a number of factors such as the amounts of the three catalysts components that are used, by-product formation, the design of the process system and the space-time yield desired.

The invention is further illustrated by the following examples.

Example 1

The reactor consisted of a lower section of five feet, five inches (1.65 m.) of two-inch (5 cm.) diameter pipe, a middle section of six feet, one inch (1.9 m) of one-inch (2.54 cm.) diameter pipe and six feet (1.82 m.) of one-half inch (1.27 cm) diameter pipe. Total reactor volume was 4.95 liters. A gas mixture of carbon monoxide and 5 volume percent hydrogen was fed to the reactor through a gas sparger at the bottom of the reactor. Through a reactor feed line, located above the sparger, was fed a mixture containing methyl acetate, acetic acid, acetic anhydride, methyl iodide, lithium and rhodium at an average rate of about 12,600 grams/hour. The reactor contents overflowed from the top of the reactor to a separator where some of the unreacted carbon monoxide and other gases were separated from the liquid and purged from the system. The liquid from the separator passed through a valve which reduced the pressure from about 750 psig (5273 kPa) to 10—20 psig (170—239 kPa). The liquid passed through a flash evaporator, wherein 80—90% of the material was vaporized and entered an evaporator separator (about 1 psig) (108 kPa) wherein the vapor and liquid were separated. The liquid was mainly acetic acid and acetic anhydride in which the rhodium and lithium catalyst components were dissolved along with minor amounts of methyl iodide and methyl acetate. It was recycled to the reactor. The vapors from the evaporator separator were fed to a column in which the temperature was maintained at about 140°C. at the base and about 100°C. at the top. Crude acetic anhydride suitable for further refining was removed from the lower portion of the column. The lower boiling components (methyl acetate, methyl iodide and some acetic acid) were taken overhead and

**0 064 986**

fed to a tank to which makeup methyl acetate was also fed. The contents of the tank were continuously fed to the reactor feed line.

Using the above-described system, acetic anhydride was produced by the carbonylation of methyl acetate at about 190°C. and 750 psig (5273 kPa). Over the course of 80 hours of continuous operation samples of the liquid from the separator and the reactor feed periodically were analyzed and the conversion of methyl acetate (methyl acetate fed minus methyl acetate in the separator liquid divided by methyl acetate fed) and the acetic anhydride space-time yield (STY, in grams/liter/hours) were determined. The data thus accumulated is shown in Table I. The hydrogen in the gas feed was stopped at hour 245 and was resumed at hour 267. As indicated by the conversion anf space-time yield data in Table I, there was no significant tar formation during the period of time when hydrogen gas was fed to the reactor.

The data of Table I show that discontinuing the hydrogen feed to the carbonylation reactor has a substantial detrimental effect on production rate, i.e. space-time yield.

TABLE I

| Operating time, hours | Reactor separator pot underflow composition | | | | | | Reactor feed | | Space-time yield (8 hour average) |
|---|---|---|---|---|---|---|---|---|---|
| | Rh ppm | Li ppm | Methyl iodide % | Methyl acetate % | Acetic acid % | Acetic anhydride % | Methyl acetate % | Acetic anhydride % | Conver-sion % | |
| 219 | 496 | 1941 | 17.7 | 28.3 | 17.6 | 34.7 | 44.6 | 15.9 | 37 | |
| 223 | | | — | — | — | — | 45.9 | 20.8 | — | |
| 227 | 504 | 3121 | 16.0 | 16.9 | 16.7 | 46.8 | 42.5 | 21.3 | 60 | 650 |
| 231 | | | 15.5 | 17.0 | 20.8 | 43.4 | 41.0 | 19.4 | 59 | |
| 235 | 790 | 3587 | 15.4 | 8.6 | 33.2 | 39.4 | 27.3 | 21.9 | 68 | 606 |
| 243 | 593 | 3206 | 19.1 | 9.9 | 23.3 | 41.9 | 25.3 | 24.1 | 61 | 482 |
| 247 | | | 27.2 | 17.8 | 27.3 | 28.9 | 20.7 | 13.5 | 14 | |
| 251 | 482 | 2802 | 21.0 | 21.4 | 26.8 | 26.3 | 37.4 | 11.9 | 43 | 254 |
| 255 | | | 19.7 | 22.4 | 30.3 | 25.7 | 45.9 | 9.5 | 51 | |
| 259 | 388 | 1634 | 25.8 | 15.4 | 32.9 | 21.8 | 38.4 | 6.9 | 60 | 292 |
| 267 | 474 | 2451 | 22.2 | 22.8 | 33.0 | 20.3 | 30.1 | 9.0 | 24 | 223 |
| 271 | | | 21.0 | 16.5 | 23.3 | 34.9 | 31.0 | 15.9 | 47 | |
| 275 | 450 | 2268 | 20.4 | 14.8 | 20.0 | 34.9 | 29.7 | 18.7 | 50 | 401 |
| 279 | | | 21.0 | 18.6 | 23.1 | 34.1 | 35.0 | 15.5 | 47 | |
| 283 | 469 | 2375 | 21.9 | 17.5 | 22.9 | 34.0 | 45.7 | 13.2 | 62 | 435 |
| 291 | 905 | 3955 | 23.4 | 24.1 | 19.9 | 31.3 | 49.7 | 8.8 | 52 | 389 |
| 295 | | | 18.6 | 24.9 | 18.9 | 36.3 | 37.5 | 16.2 | 34 | |
| 299 | 408 | 2216 | 16.7 | 17.8 | 25.9 | 35.3 | 30.9 | 17.4 | 42 | 358 |

Example 2

Four runs in which varying amounts of hydrogen were fed to the carbonylation reactor were carried out in the apparatus decribed in Example 1 except that the reactor consisted of the five feed, six inches, (1.7 m.) of pipe having a volume of 3.5 liters. The temperature was about 190°C., the pressure about 750 psig (5273 kPa) and the feed rate about 12,600 grams/hour over the course of each run. In Table II are set forth average values for each run, the composition of the feed stream (weight percent), the rhodium and lithium (ppm) present in the system, the amount of hydrogen ($H_2$) fed (volume percent of the total gas fed), the acetic anhydride space-time yield (STY; grams/liter/hour) and the weight ratio of ethylidenediacetate (EDA) to acetic anhydride ($Ac_2O$) produced. The duration of the first run was 64 hours, the second 65.5 hours, the third 73 hours and the fourth 34 hours.

In Runs 1—3, high space-time yields were obtained although the higher concentrations of hydrogen resulted in the formation of significant amounts of ethylidenediacetate. Also, in these runs, the tar formation was minimal and could be easily handled. In Run 4, essentially no ethylidenediacetate was formed but the space-time yield of acetic anhydride was decreased considerably by the use of only 1.2 volume percent hydrogen in the gas feed (less than the amount of hydrogen required by this invention).

TABLE II
Reactor feed composition

| Run | Methyl chloride | Methyl acetate | Acetic acid | $Ac_2O$ | EDA | Rh | Li | $H_2$ | STY | EDA:$Ac_2O$ |
|-----|------|------|------|------|------|-----|------|------|-----|--------|
| 1 | 15.0 | 50.4 | 20.4 | 12.8 | 0.38 | 607 | 2098 | 6.25 | 573 | 0.0131 |
| 2 | 14.8 | 51.9 | 16.8 | 15.9 | 0.39 | 696 | 1977 | 4.33 | 564 | 0.0079 |
| 3 | 16.0 | 38.8 | 27.1 | 17.7 | 0.18 | 812 | 2430 | 2.23 | 541 | 0.0033 |
| 4 | 13.1 | 38.3 | 35.2 | 13.2 | 0.04 | 817 | 2281 | 1.20 | 342 | 0 |

Example 3

Seven runs were carried out in a 1.83 liter autoclave fitted with Hastelloy B-2 baffles. Mixing was provided by a magnetic stirring bar operated at about 630 rpm. To permit obtaining liquid samples of the reaction mixture under the reaction conditions, the autoclave was equipped with a Hastelloy B-2 dip tube having the necessary valve system to permit safe removal of liquid under pressure. The temperature of the reaction mixture was monitored by an iron constantan thermocouple placed in a thermowell immersed in the liquid. Constant pressure was maintained throughout each run. The solid catalyst components and the liquid reactants were placed in the autoclave. After sealing and pressure testing, the mixture was flushed with carbon monoxide by pressurizing to 100 psig (790 kPa) with stirring followed by slowly venting to atmospheric pressure. After a second pressurization-vent cycle, the autoclave was pressurized to 10 psig (170 kPa) with the reactant gas and then heated to the desired temperature. An aliquot of the reaction mixture taken at this point was used as a zero time sample. Immediately following the initial sampling, the autoclave was pressurized to the desired value with the reactant gas. The course of the reaction was followed by removing aliquots of the reaction mixture at 30 minute intervals while maintaining reaction conditions. To ensure representative sampling, the second of two 5-ml aliquots taken at the specified sampling time was used to obtain analytical data.

The aliquot samples were cooled and analyzed directly by gas chromatography using butyronitrile as an internal standard. Data for each sample was obtained in terms of milligrams of component per gram of solution. Based on the assumption that the weight increase of the reaction mixture during the run was due only to the weight of carbon monoxide used to produce acetic anhydride ($Ac_2O$) the following formula was used to calculate the total moles of acetic anhydride present at the sampling time.

$$\text{MOLES}_{Ac_2O} = \left[ \frac{W_o}{1 - \dfrac{28}{102} X_{Ac_2O}} \right] \frac{X_{Ac_2O}}{102}$$

$W_o$ = initial weight of reaction mixture

$$X_{Ac_2O} = \frac{\text{mg}Ac_2O}{\text{gm Solution}} \times 10^{-3}$$

Runs 1 and 2 were carried out at 175°C. and 650 psig (4580/kPa) using (1) a gas feed of carbon monoxide without hydrogen and carbon monoxide containing 5 volume percent hydrogen and (2) the following materials:

7

**0 064 986**

| | |
|---|---|
| RhCl$_3$ . xH$_2$O | 1.24 g. |
| LiI | 33.85 g. |
| Acetic Acid | 220.50 g. |
| Methyl Acetate | 676.50 g. |
| CH$_3$I | 130.20 g. |

Runs 3 and 4 were conducted at 190°C. and 1000 psig., (6996 kPa) with and without hydrogen in the carbon monoxide feed using the following materials:

| | |
|---|---|
| RhCl$_3$ . xH$_2$O | 0.62 g. |
| LiI | 25.39 g. |
| Acetic Acid | 220.50 g. |
| Methyl Acetate | 676.50 g. |
| CH$_3$I | 130.20 g. |

In each of Runs 5, 6 and 7, 0, 5 and 10 volume percent hydrogen respectively, were present in the carbon monoxide fed to the autoclave. These runs were carried out at 200°C. and 1000 psig (6996 kPa) using the following materials:

| | |
|---|---|
| RhCl$_2$ . xH$_2$O | 0.30 g. |
| LiI | 12.30 g. |
| Acetic Acid | 220.50 g. |
| Methyl Acetate | 676.50 g. |
| CH$_3$I | 130.20 g. |

The results, shown in Table III, obtained from Runs 1—7 show that the inclusion of hydrogen in the carbon monoxide used to carbonylate methyl acetate substantially increased the rate at which acetic anhydride was formed. Runs 6 and 7 further show that the use of 10 volume percent hydrogen in the carbon monoxide did not increase the rate over that observed when 5 volume percent hydrogen was used. No significant quantities of the tar were formed in runs 2, 4 and 6, made according to the practice of this invention.

TABLE III

| Time, minutes | Run 1 no H$_2$ | Run 2 5% H$_2$ | Run 3 no H$_2$ | Run 4 5% H$_2$ | Run 5 no H$_2$ | Run 6 5% H$_2$ | Run 7 10% H$_2$ |
|---|---|---|---|---|---|---|---|
| 30 | 0.41 | 0.75 | 0.57 | 1.08 | 0.44 | — | 0.84 |
| 60 | 1.07 | 2.06 | 1.61 | 2.30 | 1.28 | 1.56 | 1.70 |
| 90 | 1.75 | 3.05 | 2.60 | 3.42 | 1.95 | 2.30 | 2.47 |
| 120 | 2.40 | 4.10 | 3.48 | 4.36 | 2.49 | 3.10 | 3.20 |
| 150 | 3.25 | 4.85 | 4.35 | 5.36 | 3.04 | 3.90 | 3.96 |
| 180 | 3.91 | 5.47 | 4.98 | 6.07 | 3.57 | 4.43 | 4.50 |
| 210 | 4.44 | 6.27 | 5.63 | 6.80 | 3.87 | 4.94 | 5.06 |
| 240 | 5.03 | 6.74 | 6.00 | 7.25 | 4.31 | 5.83 | 5.72 |
| 270 | 5.60 | 7.25 | 6.55 | 7.43 | 4.68 | 6.15 | 6.20 |
| 300 | 6.10 | 7.56 | 6.82 | 7.88 | — | — | — |

8

# 0 064 986

Example 4

A 300 cc autoclave was fitted with a dip tube and the necessary valve system to permit safe removal of liquid under pressure as in Example 3. The following were charged to the autoclave: $RhCl_3 \cdot xH_2O$, 0.95 g; LiI, 13.0 g; $CH_3I$, 6.10 g; acetic acid, 40.0 g; methyl acetate 144.0 g. The autoclave was sealed, pressure tested, and flushed with carbon monoxide as in Example 3. The autoclave was pressurized to 10 psig (170 kPa) with carbon monoxide and heated to 175°C. After pressurizing to 800 psig (5617 kPa) with carbon monoxide, samples are taken at 20 minute intervals. Immediately following the sampling at 120 minutes, the autoclave pressure was raised from 800 psig (5617 kPa) to 1,000 psig (6996 kPa) by the addition of hydrogen.

The total pressure was then allowed to drop to 800 psig (5617 kPa) before additional carbon monoxide was added to maintain 800 psig (5617 kPa) total pressure. The results, set forth in Table IV, show that the addition of $H_2$ substantially increased the rate of production of acetic anhydride ($Ac_2O$). In addition, no significant quantity of tar was formed over the course of the reaction.

### TABLE IV

| Time, (minutes) | $Ac_2O$ (moles) |
| --- | --- |
| 20 | 0.06 |
| 40 | 0.08 |
| 60 | 0.13 |
| 80 | 0.19 |
| 100 | 0.26 |
| 120* | 0.31 |
| 140 | 0.53 |
| 160 | 0.76 |
| 180 | 0.97 |
| 200 | 1.12 |
| 220 | 1.25 |

*$H_2$ added immediately after sample was taken.

## Claims

1. A liquid-phase process for the preparation of acetic anhydride by the carbonylation of methyl acetate in the presence of rhodium, lithium, and an iodine compound, characterized in that hydrogen gas is also fed to the reaction zone in an amount of 2 to 7 volume percent, based on carbon monoxide and hydrogen gases.

2. The process of claim 1 wherein the gas fed to the reaction zone comprises 3 to 6 volume percent of hydrogen.

3. The process of claim 1 to 2 wherein lithium is present in an amount of 175 to 5000 ppm.

4. The process of claim 3 wherein rhodium is employed in an amount of 500 to 1000 ppm, lithium is employed in an amount of 1500 to 3700 ppm, iodine in the form of methyl iodide is employed in an amount of 7 to 35 weight percent, the temperature of reaction is 160 to 220°C, and the reaction pressure is 2170 to 8375 kPa.

## Patentansprüche

1. Verfahren zur Herstellung von Essigsäureanhydrid in flüssiger Phase durch Carbonylierung von Methylacetat in Gegenwart von Rhodium, Lithium und einer Iodverbindung, dadurch gekennzeichnet, daß in die Reaktionszone auch Wasserstoffgas in einer Menge von 2 bis 7 Volumenprozent, bezogen auf Kohlenmonoxid- und Wasserstoffgase, eingespeist wird.

2. Verfahren nach Anspruch 1, dadurch gekennzeichnet, daß das in die Reaktionszone eingespeiste Gas 3 bis 6 Volumenprozent Wasserstoff enthält.

# 0 064 986

3. Verfahren nach Anspruch 1 oder 2, dadurch gekennzeichnet, daß Lithium in einer Menge von 175 bis 5000 ppm vorliegt.

4. Verfahren nach Anspruch 3, dadurch gekennzeichnet, daß Rhodium in einer Menge von 500 bis 1000 ppm, Lithium in einer Menge von 1500 bis 3700 ppm und Iod in Form von Methyliodid in einer Menge von 7 bis 35 Gew.-% verwendet werden, daß die Reaktionstemperatur bei 160 bis 220°C liegt und daß der Reaktionsdruck 2170 bis 8375 kPa beträgt.

## Revendications

1. Procédé en phase liquide pour la préparation d'anhydride acétique par carbonylation d'acétate de méthyle en présence de rhodium, de lithium et d'un composé de l'iode caractérisé en ce qu'on alimente aussi la zone de réaction avec de 2% à 7% d'hydrogène gazeux, en volume par rapport au monoxyde de carbone et à l'hydrogène gazeux.

2. Procédé de la revendication 1, dans lequel le gaz fourni à la zone de réaction comprend 3 à 6% en volume d'hydrogène.

3. Procédé de la revendication 1 ou 2, dans lequel le lithium est présent en quantité de 175 à 5000 ppm.

4. Procédé de la revendication 3, dans lequel on utilise le rhodium en quantité de 500 à 1000 ppm, le lithium en quantité de 1500 à 3700 ppm, l'iode sous forme d'iodure de méthyle en quantité de 7 à 35% en masse, la température de réaction est de 160 à 220°C et la pression de réaction est de 2170 à 8375 kPa.